# EUROPÄISCHE PATENTSCHRIFT

(11) **EP 0 523 357 B1**
(45) Veröffentlichungstag und Bekanntmachung des Hinweises auf die Patenterteilung: **03.09.1997**
(21) Anmeldenummer: 92109248.2
(22) Anmeldetag: 02.06.1992
(51) Int. Cl.: C09C 1/00

(54) **Oberflächenmodifiziertes plättchenförmiges Substrat mit verbessertem Absetz- und Aufrührverhalten**
Platy substrate with modified surface of improved properties relating to sedimentation and mixing
Substrat en forme de paillette modifié en surface aux propriétés relatives à la sédimentation et au mélange améliorées

(30) Priorität: 25.06.1991 DE 4120921
(43) Veröffentlichungstag der Anmeldung: 20.01.1993
(73) Patentinhaber: MERCK PATENT GmbH, D-64271 Darmstadt (DE)
(72) Erfinder: Herget, Gerhard, Dr., W-6105 Ober-Ramstadt (DE); Osterried, Karl, Dr., W-6110 Dieburg (DE); Glausch, Rolf, Dr., W-6100 Darmstadt 13 (DE); Maisch, Roman, Dr., W-6101 Rossdorf (DE)

(56) Entgegenhaltungen:
- GB-A- 1 238 440
- US-A- 3 549 405
- DATABASE WPI Section Ch, Week 7947, Derwent Publications Ltd., London, GB; Class A60, AN 79-84904B; & JP-A-54 131 629
- PATENT ABSTRACTS OF JAPAN, vol. 11, no. 115 (C-415), 10. April 1987; & JP-A-61 258 875

## Beschreibung

Die vorliegende Erfindung betrifft oberflächenmodifizierte Pigmente auf der Basis plättchenförmiger Substrate mit einem verbesserten Absetz- und Aufrührverhalten, sowie deren Herstellungsverfahren und Verwendung.

Beschichtungsmassen wie z.B. Lacke, Farben, Druckfarben etc., enthaltend Pigmente auf der Basis plättchenförmiger Substrate sind insofern problematisch in der Handhabung, als die Pigmente infolge ihrer Größe und ihrer Dichte sich leicht ansetzen und dann zu einem sehr festen Sedimentkuchen zusammenbacken können. Dieser Kuchen ist in der Regel nur schwer wieder aufrührbar. Dies gilt vor allem bei der Lagerung von Lacken, Farben und Druckfarben, sowie bei der Verarbeitung derselben.

So wurden unter anderem zahlreiche Methoden entwickelt, um das Problem der Einarbeitung und Handhabung von plättchenförmigen Pigmenten in Beschichtungsmassen zu lösen.

Aus der DE 36 27 329 und den EP's 0 306 056 und 0 268 918 ist bekannt, daß modifizierte plättchenförmige Substrate mit einer Polymerbeschichtung bzw. nach Behandlung mit Kupplungsreagenzien wie Organotitanaten, Organosilanen in Belegmassen ein verbessertes Absetz- und Aufrührverhalten zeigen.

Weiterhin kann das Wiederaufrühren erleichtert werden, indem den Beschichtungsmassen Additive zugesetzt werden, die entweder eine gezielte Flokkulation (Kartenhauseffekt), ein strukturviskoses und/oder thixotropes Verhalten, eine sterische Abstoßung und/oder eine elektrostatische Abstoßung der Pigmente bewirken.

Additive mit thixotropen Verhalten sind in der EP-0 198 519 und der DE-OS-18 05 693 beschrieben. Aus der DE 39 22 178 ist bekannt, daß man durch Mischen einer Suspension eines plättchenförmigen Substrats mit sphärischen Teilchen wie z.B. SiO₂, TiO₂ und ZrO₂ deagglomerierte und gut despergierbare Pigmente erhält.

Diese Zusätze können jedoch einen negativen Einfluß auf die Qualität der Beschichtung haben. Insbesondere die Brillanz bei Perlglanzpigmenten und die Gleichmäßigkeit der Beschichtung können beeinträchtigt werden. Diese Beeinträchtigungen sind im allgemeinen umso größer, je höher die Einsatzkonzentration- der Additive ist.

In der JP 54-131629 wird ein Verfahren zur Beschichtung von kubischen Calciumcarbonatpigmenten mit nadelförmigen Al₂O₃-Kristallen beschrieben.

Es bestand daher die Aufgabe oberflächenmodifizierte Pigmente auf der Basis plättchenförmiger Substrate zu finden, die bei Einarbeitung in einer Beschichtungsmasse, die bei herkömmlichen pigmentierten Beschichtungsmassen beobachteten Nachteile nicht oder nur in geringem Umfang zeigen.

Überraschenderweise wurde nun gefunden, daß Pigmente, welche auf plättchenförmigen Substraten basieren und mit einem Gemisch bestehend aus Bindemittel, faserförmigen Partikeln mit einer Faserlänge von 0,1-20 µm und einem Lösungsmittel bzw. Lösungsmittelgemisch beschichtet sind, ein verbessertes Absetz- und Aufrührverhalten in Beschichtungsmassen zeigen.

Gegenstand der Erfindung sind daher oberflächenmodifizierte Pigmente, welche auf einem plättchenförmigen Substrat basieren, die zur Verbesserung des Absetz- und Aufrührverhaltens mit einem Modifizierungsreagenz bestehend aus Bindemittel, faserförmigen Partikeln mit einer Faserlänge von 0,1-20 µm und einem Lösungsmittel bzw. Lösungsmittelgemisch beschichtet sind.

Gegenstand der Erfindung ist auch ein Verfahren zur Herstellung oberflächenmodifizierter plättchenförmiger Substrate, dadurch gekennzeichnet, daß man in einem Mischgefäß plättchenförmige Substrate mit dem Modifizierungsreagenz bestehend aus Bindemittel, faserförmigen Partikeln mit einer Faserlänge von 0,1-20 µm und einem Lösungsmittel bzw. Lösungsmittelgemisch beschichtet.

Alle bekannten plättchenförmigen Metalle, Metalloxide, Glimmerpigmente und sonstigen plättchenförmigen Substrate können nach dem erfindungsgemäßen Verfahren belegt werden.

Beispiele hierfür sind Glimmer, Talkum, Kaolin oder andere vergleichbare Mineralien sowie plättchenförmiges Eisenoxid und Wismutoxychlorid.

Da bei dem Verfahren keine hohen Scherkräfte benötigt werden, ist das Verfahren auch hervorragend zur Beschichtung von Perlglanzpigmenten geeignet.

Es können alle üblichen Perlglanzpigmente verwendet werden, z.B. Glimmerbeschichtungen mit farbigen oder farblosen Metalloxiden, wie TiO₂, Fe₂O₃, SnO₂, Cr₂O₃, ZnO und anderen Metalloxiden, allein oder in Mischung in einer einheitlichen Schicht oder in aufeinanderfolgenden Schichten. Diese Pigmente sind z.B. aus den deutschen Patenten und Patentanmeldungen 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 und 32 35 017 bekannt und im Handel erhältlich z.B. unter dem Warenzeichen Iriodin® von der Firma E. Merck, Darmstadt.

Überraschend ist auch die Beobachtung, daß die beschichteten Substrate in ihren optischen Eigenschaften nicht schlechter werden. Die Einsatzkonzentration des Modifizierungsreagenzes kann 0,1-100 Gew.% bezogen auf das Pigment betragen, vorzugsweise liegt sie jedoch bei 0,2-50 %, insbesondere bei 0,5-20 %.

Das verwendete Lösungsmittel kann auf dem Pigment verbleiben, dann stauben die als Präparation vorliegenden Pigmente nicht, oder es kann nach dem Beschichtungsprozeß entfernt werden.

Wesentlicher Bestandteil des Modifizierungsreagenzes sind die faserförmigen Partikel, die in einer Menge von etwa 0,1 bis 100 Gew.%, insbesondere 1 bis 50 Gew.%, vorzugsweise 5 bis 20 Gew.% im Modifizierungsreagenz enthalten sind. Durch Zusatz dieser sperrigen Teilchen wird verhindert, daß die nach dem erfindungsgemäßen Verfahren beschichteten Substrate sich auf Grund der sterischen Abstoßung in nennenswertem Umfang aufeinanderlegen und damit eine starke Adhäsion ausüben. Dies bewirkt, daß die beschichteten Substrate sich in Lack- und Farbsystemen zum Teil sehr viel langsamer absetzen, das Sediment aber in allen Fällen weniger hart ist, und daß keine Probleme beim Wiederaufrühren des Bodensatzes auftreten.

Alle dem Fachmann bekannten organischen und anorganischen Fasern mit einer Faserlänge von 0,1-20 µm können verwendet werden. Geeignete Partikel sind insbesondere alle Kunstfasern z. B. aus Polyethylen, Polyacrylaten, Polypropylen, Polyamiden, Cellulosefasern, anorganische Fasern hier bevorzugt Siliziumverbindungen, Glasfasern sowie insbesondere die Kondensationsprodukte aus modifizierten Isocyanaten und Mono- und Diaminen.

Diese Kondensationsprodukte, wobei es sich um Diharnstoffderivate sowie Aminoharnstoffe mit Urethangruppierungen handelt, sind als Thixotropierungsmittel bekannt und werden mit einem Bindemittel Farben und Lacken zugesetzt, um die Ablaufeigenschaften und die Streichbarkeit zu verbessern. Die Beschichtung plättchenförmiger Substrate mit einem Modifizierungsreagenz enthaltend ein Thixotropierungsmittel ist bisher nicht bekannt.

Für das Modifizierungsreagenz können alle dem Fachmann bekannten Diharnstoffderivate und Urethanverbindungen verwendet werden, wie sie z.B. in der EP 0 198 519, der DE 18 05 693.4 und in Organic Coatings: Science and Technology, A. Heenriga, P.J.G. von Hemsbergen, S. 201-222, New York 1983 beschrieben werden.

Weiterer Bestandteil des Modifizierungsreagenzes sind die Bindemittel, die in einer Menge von etwa 20 bis 50 Gew. % im Modifizierungsreagenz enthalten sind. Geeignet sind alle dem Fachmann bekannten Polymerharze mit pigmentaffinen Gruppen, die Molekulargewichte von 100-100000, vorzugsweise von 200-10000 und insbesondere von 500-5000 aufweisen, z. B. Alkyl-, Acryl-, Alkyd-, Acrylatharze, Polyester sowie Mischungen aus mehrerer Polymerharzen.

Das erfindungsgemäße Verfahren ist einfach und läßt sich leicht handhaben. Die Herstellung der Präparation geschieht durch Vermischen der genannten Wirksubstanzen in einem Lösungsmittel. Geeignet sind alle organischen Lösungsmittel z. B. Ester, Acetate, Alkohole wie Ethanol, Propanol, Isopropanol, Methoxypropanol, tert.-Butanol, sowie aromatische Lösungsmittel z. B. Benzol, Xylol, insbesondere Toluol. Das Modifizierungsreagenz ist dadurch gekennzeichnet, daß es 5 bis 80 Gew.%, insbesondere 10 bis 70 Gew.% an Lösungsmittel bzw. Lösungsmittelgemisch enthält.

Das Aufbringen des Modifizierungsreagenzes auf das Pigment erfolgt durch einfaches Mischen mit dem Substrat z. B. in einem Taumel-, Flügelrad-, Schaufel- oder Fluidmischer, wobei auf Grund der relativ hohen Bruchanfälligkeit der Substrate langsam laufende Mischer bevorzugt werden. Da keine hohen Scherkräfte bei der Pigmentpräparation notwendig sind, ist das erfindungsgemäße Verfahren auch für Perlglanzpigmente hervorragend geeignet.

Die erfindungsgemäße Pigmentpräparation ist mit einer Vielzahl von Farbsystemen kompatibel vorzugsweise aus dem Bereich der Lacke, Farben und Druckfarben.

Gegenstand der Erfindung ist somit auch die Verwendung der beschichteten Substrate in Formulierungen wie Farben, Druckfarben, Lacken und zur Kosmetikpräparation.

Zur Erläuterung der Erfindung dienen die folgenden Beispiele:

### I. Untersuchungen des Absetz- und Aufrühverhaltens der Pigmentpräparationen in Druckfarben.

### Beispiel 1

In einem Mischgefäß mit Flügelradrührer werden 200 g Iriodin 100 (TiO₂-Glimmer mit einer Teilchengröße von 10-60 µm von der Fa. E. Merck, Darmstadt) vorgelegt und unter Rühren mit einer Mischung aus 20 g Setal 90173 SS-50 in 40 g 1-Methoxy-2-propanol (Modifizierungsreagenz bestehend aus einem Diharnstoffderivat, Polyesterharz, Xylol, Solvesso 100 der Fa. Synthese, Boxmer sowie 1-Methoxy-2-propanol über einen Zeitraum von 5 Minuten versetzt.

Man erhält eine nichtstaubende Pigmentpräparation mit hervorragendem Redispergierverhalten.

### Beispiel 2

Analog Beispiel 1 wird Iriodin 300 (Fe₂O₃/TiO₂-Glimmer mit einer Teilchengröße von 10-60 µm von der Fa. E. Merck, Darmstadt) mit 20 g Setal belegt.

### Beispiel 3

Analog Beispiel 1 wird Iriodin 500 (Fe₂O₃-Glimmer mit einer Teilchengröße von 10-60 µm von der Fa. E. Merck, Darmstadt) mit 20 g Setal belegt.

### Beispiel 4 a

Analog Beispiel 1 wird Iriodin 225 (TiO₂-Glimmer mit einer Teilchengröße von 10-60 µm von der Fa. E. Merck, Darmstadt) mit 20 g Setal belegt.

### Beispiel 4 b

Analog Beispiel 1 werden 200 g Iriodin 225 vorgelegt und mit 10 g Setal versetzt.

### Beispiel 4 c

Analog Beispiel 1 werden 200 g Iriodin 225 vorgelegt und mit 5 g Setal versetzt.

### Beispiel 5

45 g eines kommerziell erhältlichen Nitrocellulose-Kombinationsverschnittes der Fa. Huber München, Kirchheim-Heimstetten, werden unter Rühren mit einem Flügelrührer portionsweise mit 15 g Iriodin 100 versetzt. Mit einer 1:1-Mischung aus Ethylacetat/Ethanol wurde auf 17 sec Auslaufzeit eingestellt (DIN 4-Becher).

### Beispiel 6

Analog Beispiel 5 werden 45 g des Nitrocellulose-Kombinationsverschnittes unter Rühren portionsweise mit 19,5 g Iriodinpräparation (Beispiel 1) versetzt. Mit einer 1:1-Mischung Ethylacetat/Ethanol wird auf 17 sec Auslaufzeit eingestellt.

### Beispiel 7

### Absetz- und Redispergierversuche

Jeweils 50 ml der pigmentierten Druckfarben der Beispiele 5 und 6 werden in einen Meßzylinder gefüllt. Über 30 Tage wird das Sedimentvolumen bestimmt. Mit einem speziellen Meßdorn wird am 30. Tag die Eindringtiefe bestimmt im Verhältnis zur gesamten Sedimenthöhe. Die Eindringtiefe ist ein Maß für die Festigkeit des Sediments und verhält sich umgekehrt wie diese.

**Tabelle**

| | Sedimentvolumen (ml) | | | | | | Eindringtiefe/Sedimenthöhe (mm/mm) |
|---|---|---|---|---|---|---|---|
| | 1 | 2 | 5 | 24 | 48 | 30 Tage | |
| Beispiel 5 | - | 47.5 | 45 | 17 | 17 | 13.2 | 15/35 |
| Beispiel 6 | - | 49 | 46.5 | 30.2 | 27.5 | 19 | 51/51 |

Tiefdruck-Handmuster mit beiden Druckfarben unterscheiden sich nicht im Glanzverhalten.

### Beispiel 8

45 g einer handelsüblichen Druckfarbe auf Nitrocellulosebasis der Fa. Hartmann, Frankfurt, wurden mit 15 g Iriodin 100 bzw. 300 bzw. 500 unter Rühren versetzt. Mit Ethylacetat/Ethanol 1:1 wurde auf 18 sec DIN 4-Becher eingestellt.

### Beispiel 9

45 g einer handelsüblichen Druckfarbe auf Nitrocellulosebasis der Fa. Hartmann, Frankfurt, wurden mit 19,5 g Pigmentpräparation aus Beispiel 1, bzw. Beispiel 2, bzw. Beispiel 3 versetzt. Mit Ethylacetat/Ethanol wurde auf 18 sec DIN 4-Becher eingestellt.

### Beispiel 10

Absetz- und Redispergierversuche entsprechend Beispiel 7

| | Sedimentvolumen (ml) nach Standzeit (h) | | | | | Eindringtiefe/Sedimenthöhe (mm/mm) |
|---|---|---|---|---|---|---|
| | 1 | 5 | 24 | 4 Tage | 40 | |
| Beispiel 8 (Iriodin 100) | 49 | 40 | 24 | 24.2 | 20 | 54*/54 |
| Beispiel 1 | 4.8 | 40.2 | 35.2 | 34 | 29 | 78 /78 |
| Beispiel 8 (Iriodin 300) | 49.8 | 40 | 20.5 | 20.8 | 16 | 25 /43 |
| Beispiel 1 | 48 | 48 | 26 | 25.5 | 23.2 | 62*/62 |
| Beispiel 8 (Iriodin 500) | 48.5 | 41.5 | 23.5 | 23.8 | 19 | 51*/51 |
| Beispiel 1 | 49.5 | 46.5 | 31 | 28 | 25.8 | 69 /69 |

| | | | | | | |
|---|---|---|---|---|---|---|
| * Der Dorn gleitet langsam bis zum Boden. | | | | | | |

Die Tiefdruck-Handmuster aller drei Druckfarben mit Iriodin-Präparation hatten die gleiche Brillanz wie die Vergleichsmuster.

### Beispiel 11

Jeweils 45 g eines Nitrocelluloseverschnitts (bestehend aus 9,62 % Nitrocellulose, 3,85 % Hartharz und 86,53 % Ethanol; Feststoffgehalt=13,46 %) wurden unter Rühren mit Flügelrührer mit 15 g Iriodin 225 bzw. 19,5 g Präparation 4 a), bzw. 17,3 g Präparation 4 b), bzw. 16,1 g Präparation 4 c) portionsweise versetzt. Mit 1-Methoxy-2-propanol wurde auf 16 sec DIN 4-Becher eingestellt.

### Beispiel 12

Absetz- und Redispergierversuche entsprechend Beispiel 7

**Tabelle**

| Beispiel 11 | Sedimentvolumen (ml) nach Standzeit (h) | | | | | Eindringtiefe/Sedimenthöhe (mm/mm) |
|---|---|---|---|---|---|---|
| | 2 | 8 | 24 | 4 Tage | 8 | |
| Iriodin 225 | 45 | 30 | 10 | 10 | 10 | 7/27 |
| Präparation 2,5 % Modifizierungsreagenz | 40 | 18 | 11 | 11 | 11 | 8/29 |
| Präparation 5 % Modifizierungsreagenz | 40 | 15 | 14 | 14 | 14 | 37/37 |
| Präparation 10 % Modifizierungsreagenz | 41.8 | 24 | 20 | 20 | 20 | 53/53 |

### II. Untersuchung des Absetz- und Aufrührverhaltens der Pigmentpräparationen in Lacken.

2.1 Versuchsdurchführung
Das Pigment wird modifiziert und in verschiedene Lacksysteme eingearbeitet. Der gesamte Ansatz wird in einem 50 ml Meßzylinder bis zu einer Höhe von 30 ml eingefüllt und das Absetzen des Pigments in ml in wöchentlichem Abstand registriert.
2.2 Lackensätze
a) Vergleichsansatz
   Im Vergleichsansatz werden 5 % unbelegtes Substrat auf die gesamte Lackformulierung in verschiedenen Lacksystemen eingearbeitet und das Absetzverhalten ermittelt.
b) Additionszugabe in Lack
   Der Ansatz wird wie unter a) hergestellt und zusätzlich werden 10 % Setal bezogen auf das Pigmentgewicht dem Lacksystem zugesetzt und Absetzverhalten ermittelt.
c) Zugabe des modifizierten Pigments
   Das erfindungsgemäße Pigment wird wie unter a) in das Lacksystem eingearbeitet und das Absetzverhalten ermittelt.

2.3 Ergebnisse
a) Absetzverhalten 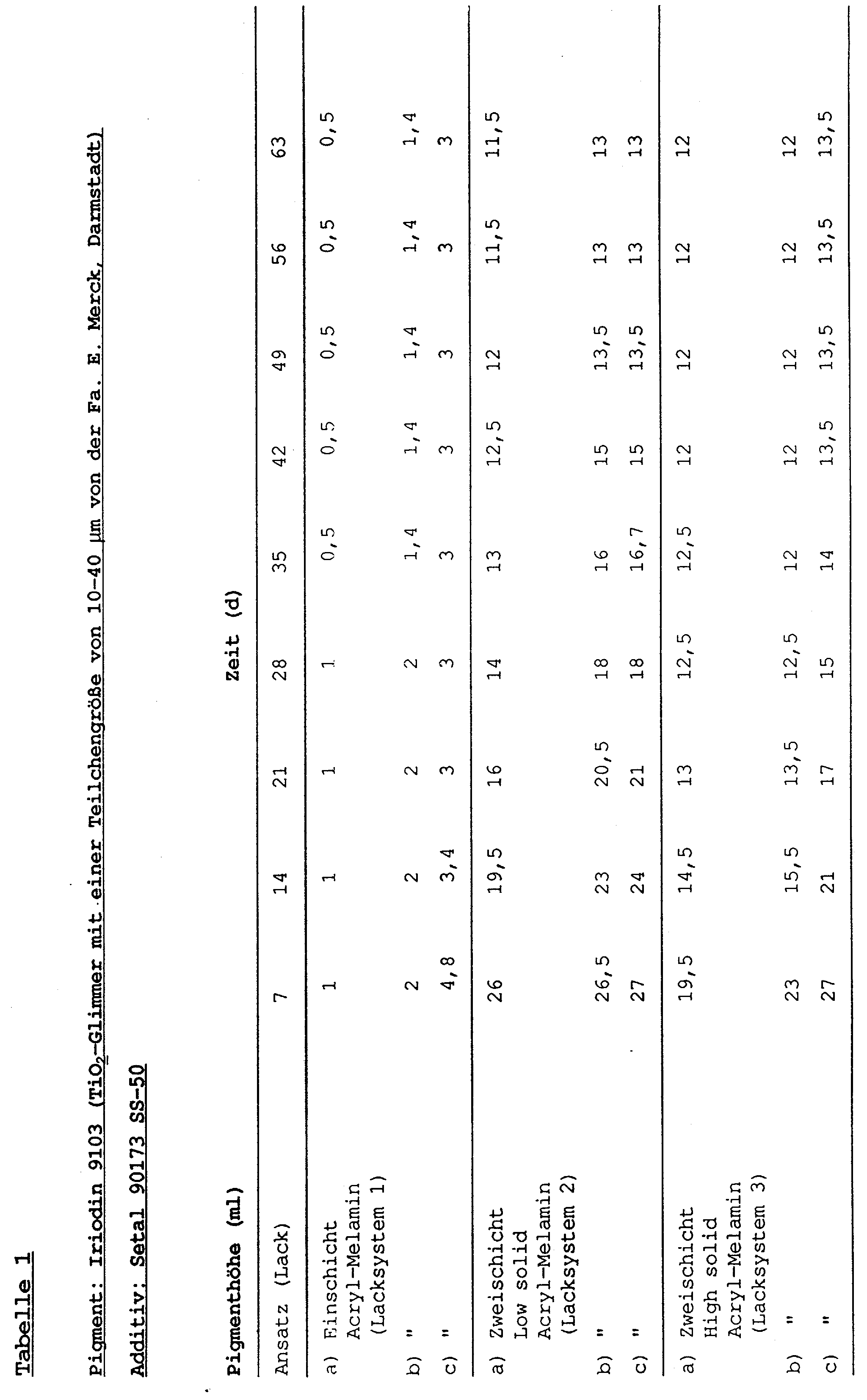
Wie aus Tabelle 1 ersichtlich, ist die Höhe des Pigments in jedem Fall bei den Ansätzen c) am größten, d. h. die Pigmente setzen sich weniger als die aus a) und b).
Gleiche Ergebnisse wurden mit Iriodin 504 (Fe₂O₃-Glimmer mit einer Teilchengröße von 10-60 µm von der Fa. E. Merck, Darmstadt) erzielt.
b) Wiederaufrührbarkeit des Bodensatzes 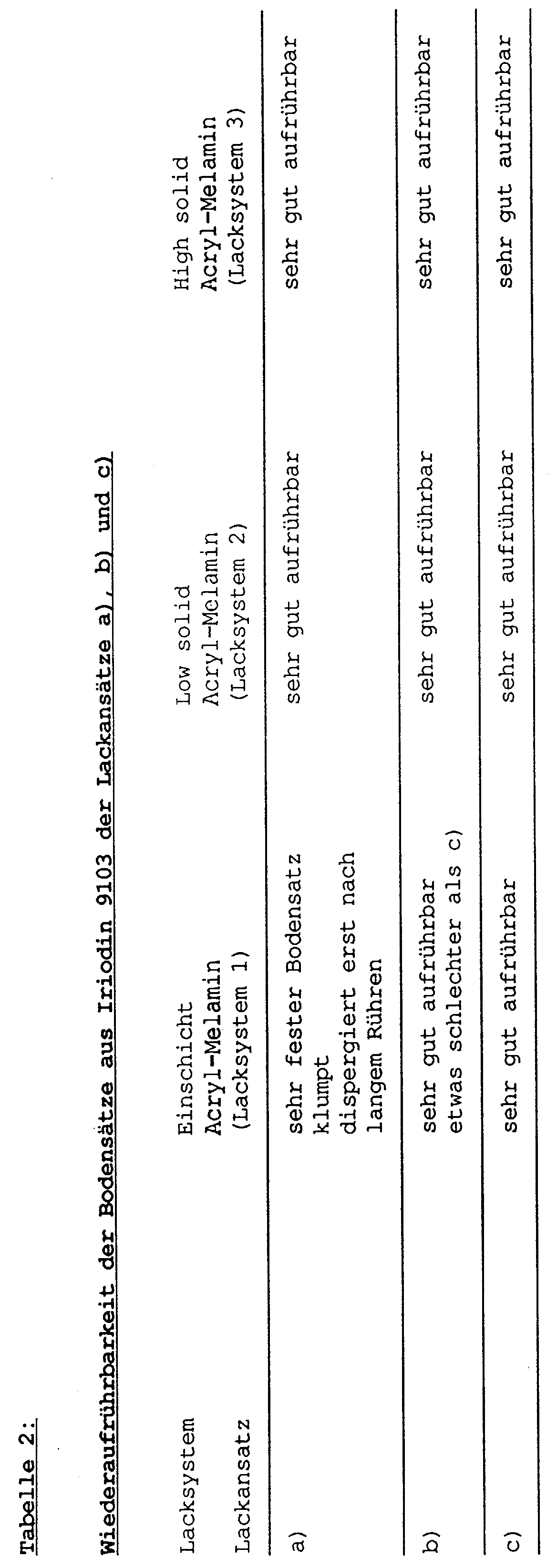
Analog 3 a ist der Lackansatz c) in jedem Falle am leichtesten aufrührbar.
c) Glanzmessung
Es wurde der Einfluß der organischen Beschichtung auf den Glanz ermittelt (Glanzmeßsystem nach Erichsen) und kein signifikanter Einfluß bei den beiden Zweischichtsystemen (Lacksysteme 2 + 3) festgestellt. Beim Einschichtsystem (Lacksystem 1) wurde durch die organische Beschichtung eine Glanzerhöhung erreicht.

**Tabelle 3**

| Lacksystem | Lackansätze (s. 2.2.) | Erichsen |
|---|---|---|
| Einschicht Acryl-Melamin (Lacksystem 1) | a) | 18 |
| | b) | 35 |
| | c) | 59 |
| Zweischicht Low solid Acryl-Melamin (Lacksystem 2) | a) | 98 |
| | b) | 97 |
| | c) | 95 |
| Zweischicht High solid Acryl-Melamin (Lacksystem 3) | a) | 93 |
| | b) | 95 |
| | c) | 94 |

## Patentansprüche

1. Oberflächenmodifizierte Pigmente, welche auf plättchenförmigen Substraten basieren, die zur Verbesserung des Absetz- und Aufrührverhaltens mit einem Modifizierungsreagenz bestehend aus Bindemittel, faserförmigen Partikeln mit einer Faserlänge von 0,1-20 µm und einem Lösungsmittel bzw. Lösungsmittelgemisch beschichtet sind.

2. Verfahren zur Herstellung oberflächenmodifizierter plättchenförmiger Substrate nach Anspruch 1, dadurch gekennzeichnet, daß man in einem Mischgefäß plättchenförmige Substrate mit dem Modifizierungsreagenz bestehend aus Bindemittel, faserförmigen Partikeln mit einer Faserlänge von 0,1-20 µm und einem Lösungsmittel bzw. Lösungsmittelgemisch beschichtet.

3. Verfahren nach Anspruch 2, dadurch gekennzeichnet, daß es sich bei den faserförmigen Partikeln um Kunststoffasern, anorganische Fasern, Cellulosefasern, Kondensationsprodukte aus Isocyanaten und Mono- bzw. Diaminen handelt.

4. Verfahren nach Anspruch 2, dadurch gekennzeichnet, daß es sich bei dem Bindemittel um Polymerharze handelt.

5. Verfahren nach Anspruch 4, dadurch gekennzeichnet, daß das Bindemittel Molekulargewichte zwischen 100 und 100 000 aufweist.

6. Verfahren nach Anspruch 2, dadurch gekennzeichnet, daß als Lösungsmittel aromatische Lösungsmittel, Alkohole, Alkylacetate sowie deren Mischungen verwendet werden.

7. Verwendung der Substrate nach Anspruch 1 in Formulierungen wie Farben, Lacken, Druckfarben, Kunststoffen und zur Kosmetikpräparation.

8. Formulierungen enthaltend Substrate nach Anspruch 1.

## Claims

1. Surface-modified pigments based on flaky substrates which to improve the settling and redispersing characteristics have been coated with a modifying agent comprising a binder, fibrous particles having a fibre length of 0.1-20 µm and a solvent or solvent mixture.

2. Process for preparing surface-modified flaky substrates according to Claim 1, characterised in that flaky substrates are coated with the modifying agent comprising a binder, fibrous particles having a fibre length of 0.1-20 µm and a solvent or solvent mixture in a mixing vessel.

3. Process according to Claim 2, characterised in that the fibrous particles are synthetic fibres, inorganic fibres, cellulose fibres, condensation products of isocyanates and mono- and diamines.

4. Process according to Claim 2, characterised in that the binder is a polymer resin.

5. Process according to Claim 4, characterised in that the binder has molecular weights between 100 and 100,000.

6. Process according to Claim 2, characterised in that the solvent used is an aromatic solvent, an alcohol, an alkyl acetate or a mixture thereof.

7. Use of the substrates according to Claim 1 in formulations such as paints, lacquers, printing inks, plastics and for making cosmetics.

8. Formulations containing substrates according to Claim 1.

## Revendications

1. Pigments à surface modifiée qui sont à base de substrats en forme de plaquettes, qui pour améliorer leur comportement de sédimentation et de dispersion sont recouverts d'un réactif modificateur constitué d'un liant, de particules en forme de fibres ayant une longueur de fibre de 0,1-20 µm et d'un solvant ou mélange de solvants.

2. Procédé de préparation de substrats en forme de plaquettes à surface modifiée selon la revendication 1, caractérisé en ce que dans un récipient de mélange on recouvre les substrats en forme de plaquettes avec un réactif modificateur constitué d'un liant, de particules en forme de fibres ayant une longueur de fibres de 0,1-20 µm et d'un solvant ou mélange de solvants.

3. Procédé selon la revendication 2, caractérisé en ce que, pour les particules en forme de fibres, il s'agit de fibres synthétiques, de fibres inorganiques, de fibres de cellulose, de produits de condensation d'isocyanates et de mono- ou de diamines.

4. Procédé selon la revendication 2, caractérisé en ce qu'il s'agit, en ce qui concerne le liant, de résines polymères.

5. Procédé selon la revendication 4, caractérisé en ce que le liant présente un poids moléculaire compris entre 100 et 100 000.

6. Procédé selon la revendication 2, caractérisé en ce qu'on utilise comme solvant des solvants aromatiques, des alcools, des acétates d'alkyle ainsi que leurs mélanges.

7. Application des substrats selon la revendication 1 à des formulations comme les colorants, les vernis, les encres d'imprimerie, les matières plastiques et à la préparation des cosmétiques.

8. Formulations contenant des substrats selon la revendication 1.
